# EUROPÄISCHE PATENTSCHRIFT

(11) **EP 1 078 625 B1**
(45) Veröffentlichungstag und Bekanntmachung des Hinweises auf die Patenterteilung: **22.06.2005**
(21) Anmeldenummer: 00250270.6
(22) Anmeldetag: 10.08.2000
(51) Int. Cl.: A61K 7/00, A61K 7/027

(54) **Körperpflegemittel in Form eines frei stehenden Stiftes**
Personal care product in the shape of a free standing stick
Produit d'hygiene corporelle en forme de baton autoportant

(30) Priorität: 19.08.1999 DE 19940221
(43) Veröffentlichungstag der Anmeldung: 28.02.2001
(73) Patentinhaber: Coty B.V., 2031 CC Haarlem (NL)
(72) Erfinder: Barone, Salvatore, Staten Island, New York 10314 (US); Yu, Wei H., Montclair, NJ 07042 (US); Kulkarni, Rupali, Bridgewater, NJ 08807 (US); Macchio, Ralph, Sparta, NJ07971 (US); Corrigan, Antonietta, Somerville, NJ 08876 (US)
(74) Vertreter: Walter, Wolf-Jürgen

(56) Entgegenhaltungen:
- WO-A-98/08484
- WO-A-98/19652
- GB-A- 2 014 852
- US-A- 4 980 192
- PATENT ABSTRACTS OF JAPAN vol. 010, no. 235 (C-366), 14. August 1986 (1986-08-14) & JP 61 068405 A (SHISEIDO CO LTD), 8. April 1986 (1986-04-08)

## Beschreibung

Die Erfindung betrifft ein neues kosmetisches Körperpflegemittel sowie Mittel zur Verschönerung des Körpers, wobei das Mittel als frei stehender Stift vorliegt.

Es sind eine Reihe kosmetischer Produkte bekannt, die in Stiftform auf dem Markt sind, wie Lippenstifte, Deostifte, Pflegestifte usw. Allen diesen Stiften ist gemeinsam, daß sie im wesentlichen auf Wachsbasis aufgebaut sind, um die entsprechende Standfestigkeit bei Umgebungstemperatur zu erreichen. Nachteilig bei diesen Produkte ist oftmals deren Klebrigkeit und/oder die unerwünschte Übertragung von Farbstoffen z.B. bei Lippenstiften auf Hautpartien, wo eine Färbung nicht erwünscht ist, z.B. auf die Finger.

GB-A-20 14 852 offenbart Lippenstifte mit kern und wachshaltiger Hülle.

Der Erfindung liegt die Aufgabe zugrunde, einen frei stehenden kosmetischen Stift als Körperpflegeprodukt zu entwickeln, dessen Außenfläche keine Farbkörper bei kurzer Berührung mit der Haut überträgt und der auch nicht klebrig ist.

Erfindungsgemäß ist das neue Körperpflegemittel in Form eines frei stehenden Stiftes dadurch gekennzeichnet, daß es aus einem Innenkern mit kosmetischen Bestandteilen und einer an dem Innenkern haftenden, festen äußeren Umhüllung besteht, wobei die äußere Umhüllung wachsfrei ist und wenigstens die folgenden Bestandteile enthält, bezogen auf die Zusammensetzung der äußeren Umhüllung,
(a) ein oder mehrere feste Proteine mit einem Anteil von 0,5 bis 50 Gew-%;
(b) ein oder mehrere flüssige Polyole oder eine Lösung von Polyolen mit einem Anteil von 0,2 bis 15 Gew-%;
(c) ein oder mehrere feste Polyole mit einem Anteil von 0,5 bis 80 Gew-%;
(d) ein oder mehrere Erweichungsmittel mit einem Anteil von 1 bis 90 Gew-%;
(e) weitere kosmetische Hilfsstoffe und aktive Ingredienzien mit einem Anteil von 0 bis 80 Gew-%.

Als feste Proteine für die äußere Umhüllung werden Proteine eingesetzt, die Protein aus der Gruppe einschließen, bestehend aus Sojaprotein, Milchprotein, Weizenprotein, Seidenprotein, Erbsenprotein, Weizenprotein, Placentaprotein, Kollagen, Reisprotein, pflanzliches Protein, Weizengluten, Weizenkeimprotein, Molkenprotein, Kartoffelprotein, Süßmandelprotein, Reisschalenprotein, Hefeprotein, Keratin, Maisprotein, Markprotein, Conchiolin-Protein, Elastin, Serumprotein und Derivate davon, einschließlich hydrolysierte Derivate, Esterderivate, hydrierte Derivate sowie Gemische dieser Proteine.

Der Gehalt dieser festen Proteine liegt vorzugsweise im Bereich von 5 bis 50 Gew-%, insbesondere 10 bis 50 Gew-%. Ein bevorzugterer Bereich liegt bei 20 bis 40 Gew-%.

Als flüssige Polyole können solche Stoffe eingesetzt werden, die geradkettige oder verzweigte Kohlenwasserstoffverbindungen mit wenigstens zwei Hydroxygruppen am Kohlenstoffgerüst einschließen. Dies sind beispielsweise Sorbitol, Propylenglycol, Arachidylglycol, Benzylglycol, Butylenglycol, Butoxydiglycol, C14-18-Glycol, C15-18-Glycol, C18-30-Glycol, C20-30-Glycol, Caprylylglycol, Ceteareth-60-myristylglycol, Cetylglycol, Diethylenglycol, Dimethoxyglycol, Dipropylenglycol, Ethoxydiglycol, Glycol, Hexacosylglycol, Hexylenglycol, Hydrogenated Talloweth-60 Matristylglycol, Laurylglycol, Methoxydiglycol, Neopentylglycol, Octacosanylglycol, PEG-10-Propylenglycol, Pentylenglycol, PEG-75-PEG-300-Hexylenglycol, Stearylglycol, Thiodiglycol, Triethylenglycol, Tripropylenglycol, Glycerin; und Gemische davon.

Die Menge dieser flüssigen Polyole oder Lösungen von Polyolen liegt insgesamt vorzugsweise im Bereich von 1,0 bis 10 Gew-%.

Weiterhin werden feste Polyole für die äußere Umhüllung bei der Zusammensetzung der vorliegenden Erfindung verwendet. Polyole sind Verbindungen, die drei oder mehr Hydroxygruppen pro Molekül enthalten einschließlich der Polysaccharide. Als feste Polyole können solche Substanzen verwendet werden, zu denen Diglycerin, Fructose, Glucose, Inositol, Lactitol, Lactose, Maltitol, Maltose, Mannitol, Sorbitol, Saccharose, Polyglycerin, Stärken wie Maisstärke, Sojastärke, Weizenstärke, Haferstärke, Kartoffelstärke, Reisstärke, Maniokstärke; und modifizierte Stärke sowie deren Derivate; Cellulose und Cellulosederivate; und Gemische davon.

Die Menge dieser festen Polyole liegt insgesamt vorzugsweise im Bereich von 15 bis 70 Gew-%, vorzugsweise 25 bis 70 Gew-%. Ein weiterer spezieller Bereich ist 30 bis 70 Gew-% oder 2 bis 40 Gew-%.

Als Erweichungsmittel (emollients) können solche eingesetzt werden, wie Fette, Öle, Fettalkohole, Fettsäureether und - ester sowie Gemische davon. Beispiele dafür sind Arecocoabutter, Shea-Butter, Ziegenbutter, Illipebutter, Jojobabutter, Mangobutter, Avocadobutter, Shorea sponoptera-Butter, Pentadesma butytacea-Butter, Pflanzenöl, Rizinusöl, Baumwollsamenöl, Koskosnußöl, C12-18-Triglyceride, Jojobaöl, Lanolin, Menhadennöl, Milchlipide, Minköl, Olivenöl, Orangen-Rohöl; Palmkernöl, Palmöl, Erdnußöl, Rapssamenöl, Sojabohnenöl, Haifischleberöl sowie deren Glycerylester und Derivate, wie hydrierte öle sowie Gemisch der genannten Substanzen.

Die Menge an Erweichungsmitteln liegt vorzugsweise im Bereich von 2 bis 40 Gew-%.

Außerdem kann die äußere Umhüllung Farbkörper und Farbstoffe enthalten, wie färbende Stoffe und übliche Pigmente. Bevorzugte Pigmente sind Calcium-, Barium- und Aluminiumschuppen, Eisenoxide, Titaniumoxid und Glimmer.

Weiterhin kann die äußere Umhüllung aktive Hautpflegebestandteile und gegebenenfalls weitere Bestandteile enthalten, wie Antioxidationsmittel, UV-Filter, Feuchthaltemittel, Schutzmittel, Parfüm usw.

Der Innenkern des Körperpflegemittels enthält Wachs, Erweichungsmittel, aktive Hautpflegebestandteile und gegebenenfalls weitere Bestandteile, wie Farbstoffe, Pigmente, Antioxidationsmittel, UV-Filter, Feuchthaltemittel, Schutzmittel, Parfüm und solche Begleitstoffe, die dem Fachmann auf diesem Gebiet bekannt sind.

Als Wachs für den Innenkern kann eingesetzt werden ein Wachs, ausgewählt aus der Gruppe, bestehend aus Candelillawachs, Bienenwachs, Carnaubawachs, Spermacetiwachs, Montanwachs, Ozokerit, Ceresin, Paraffin, modifiziertes Bienenwachs, Myricawachs, Rizinuswachse, synthetische Wachse, mikrokristalline Wachse sowie Gemische davon.

Die Menge der Wachse liegt im Bereich von 5 bis 90 Gew-%, vorzugsweise von 10 bis 30 Gew-%, am bevorzugtesten bei 10 bis 20 Gew-%, bezogen auf die Zusammensetzung des inneren Kernes.

Die Erweichungsmittel des inneren Kernes können die gleichen sein wie in üblichen Stiften oder wie in der äußeren Umhüllung. Erweichungsmittel sind Hautkonditioniermittel, einschließlich Erweichungsmittel an sich, Feuchthaltemittel, Einschlußmittel (Okklusiva) und andere diverse Begleitstoffe, die die Haut konditionieren im Sinne der CTFA-Regeln. Die Menge an Erweichungsmitteln im inneren Kern liegt im Bereich von 1 bis 90 Gew-%, vorzugsweise 10 bis 80 Gew-%, bevorzugter 20 bis 70 Gew-% und am bevorzugtesten im Bereich von etwa 40 bis etwa 60 Gew-%., bezogen auf die Zusammensetzung des inneren Kernes.

Die aktiven Hautpflegebestandteile, die wasserlöslich oder wasserunlöslich sind, können den Stiften zugesetzt werden. Zu diesen Ingredienzen gehören fettlösliche Vitamine, wie Vitamin A und E, Sonnenschutzmittel und pharmazeutisch wirksame Ingredienzen. Zu den aktiven Hautpflegeingredienzen gehören Zinkoxid, Kamillenöl, Ginkgo biloba-Extrakt, Proglutaminsäure -salze oder -ester; Natriumhyaluronat; 2-Hydroxyoctansäure; Schwefel; Salicylsäure; Carboxymethylcystein; sowie Gemische davon. Die gleichen Hautpflegeingredienzen können in der äußeren Umhüllung eingesetzt werden.

Die Menge der Hauptpflegeingredienzen im inneren Kern liegt im Bereich von 0,0001 bis etwa 10 Gew-%, bezogen auf die Zusammensetzung des inneren Kernes. Die gleiche Menge ist auch in der äußeren Umhüllung einsetzbar.

Die Menge der Farbstoffe im inneren Kern liegt im Bereich von 0 bis etwa 35 Gew-%, vorzugsweise von etwa 1 bis etwa 20 Gew-%, bezogen auf die Zusammensetzung des inneren Kernes. Die gleiche Menge ist auch in der äußeren Umhüllung einsetzbar.

Die Dicke der äußeren Umhüllung im Verhältnis zum inneren Kern liegt bei etwa 0,1 bis 1 zu 1, vorzugsweise etwa 0,2 bis etwa 0,5, bezogen auf den Radius des inneren Kernes.

Das neue Körperpflegemittel mit der äußeren Umhüllung ohne Wachsbasis hat eine feste Oberfläche, fließt auch bei höheren Temperaturen nicht und haftet daher auch nicht an den Fingern bei kurzer Berührung. Entsprechend den weiteren Bestandteilen hat es Anti-Alterungswirkung, ist lindernd, feuchthaltend und bietet für die Haut Nährstoffe an.

Es wurde gefunden, daß nur das Vorhandensein der Proteine zusammen mit Polyolen und Fetten/Ölen zu einem Produkt mit steifer Konsistenz führt, das zu einem frei stehenden Stift führt.

Das Körperpflegemittel kann als Lippenstift, Gesichtsstift, Augenstift und Körperstift verwendet werden, vorzugsweise als Lippenstift.

Ein besonderes Merkmal der Erfindung ist die Wärmebeständigkeit der äußeren Umhüllung, die sich insbesondere darin äußert, daß der Tropfpunkt der äußeren Umhüllung oberhalb 105 °C liegt, während die Tropfpunkte üblicher kosmetischer Stifte etwa 60 °C betragen.

Die Herstellung eines Stiftes mit Innenkern erfolgt auf bekannte Weise, zum Beispiel mit einer Gießanlage gemäß EP-B-0712593.

Die Erfindung soll nachstehend durch Beispiele näher erläutert werden. Alle Angaben erfolgen in Gewichtsprozent, sofern nichts anderes angegeben ist.

| Beispiel 1 | |
|---|---|
| **Äußere Umhüllung** | |
| **Phase A** | |
| Sheabutter | 18 |
| Hydriertes Palmöl | 8 |
| Palmöl | 10 |
| Rizinusöl | 13 |
| Sojaprotein | 15 |
| Modifizierte Maisstärke | 10 |
| Nylon-12 | 4 |
| Sericite GMS-4C | 15 |
| Pigment | 6 |

| **Phase B** | |
|---|---|
| Glycerin | 1 |

| Beispiel 2 | |
|---|---|
| **Äußere Umhüllung** | |
| **Phase A** | |
| Illipebutter | 20 |
| Hydriertes Palmöl | 6 |
| Polyisobutylen | 5 |
| Tridecyl trimellitate | 10 |
| PEG-4 Diheptanoate | 4 |
| Isopropylpalmitate | 6 |
| Rizinusöl | 8 |
| Sojaprotein | 15 |
| Cellulose PH-105 | 10 |
| Sericite GMS-4C | 10 |
| Pigment | 5 |

| **Phase B** | |
|---|---|
| Glycerin | 1 |

| Beispiel 3 | |
|---|---|
| **Äußere Umhüllung** | |
| **Phase A** | |
| Illipe butter | 27 |
| Sojaprotein | 15 |
| Lauroyl lysine | 1 |
| Nylon-12 | 10 |
| Seidenglimmer | 10,5 |
| Pigment | 10 |
| Rizinusöl | 20 |

| **Phase B** | |
|---|---|
| Glycerin | 6,5 |

| Beispiel 4 | |
|---|---|
| **Äußere Umhüllung** | |
| **Phase A** | |
| Shea butter | 26 |
| Hydriertes Palmöl | 7,5 |
| Palmöl | 7,5 |
| Rizinusöl | 15 |
| Seidenglimmer | 10 |
| Modifzierte Maisstärke | 10 |
| Seidenpulver | 15 |
| Pigment | 5 |

| **Phase B** | |
|---|---|
| Glycerin | 4 |

| Beispiel 5 | |
|---|---|
| **Äußere Umhüllung** | |
| **Phase A** | |
| Shea butter | 20 |
| Hydriertes Palmöl | 8 |
| Palmöl | 10 |
| Rizinusöl | 7 |
| Hydriertes Rizinusöl | 1 |
| Cetearyl methicone | 4 |
| Sojaprotein | 15 |
| Reisstärke | 13 |
| Sericite GMS-4C | 10 |
| Pigment | 5 |

| **Phase B** | |
|---|---|
| Glycerin | 7 |

| Beispiel 6 | |
|---|---|
| **Innerer Kern** | |
| **Phase A** | |
| Candelilla | 2 |
| C20-40 Alcohol | 3 |
| Hydriertes Rizinusöl | 4 |
| PEG 400 Bienenwachs | 4 |
| Cetyl alcohol | 3 |
| Petrolatum | 4 |
| Silica | 1 |
| Palmöl | 4 |
| Polyethylen | 2 |
| Caprylic/Capric triglycerides | 10 |
| Octyl dodecanol | 12 |
| Triglycerol diisostearate | 2 |
| Rizinusöl | 12 |
| Reisstärke | 15 |
| Keratinpulver | 10 |
| Pigmente | 10 |

| **Phase B** | |
|---|---|
| Glycerin | 2 |

| Beispiel 7 | |
|---|---|
| **Innerer Kern** | |
| **Phase A** | |
| Candelilla | 2 |
| C20-40 Alcohols | 3 |
| Hydriertes Rizinusöl | 4 |
| PEG 400 Bienenwachs | 4 |
| Cetyl alcohol | 3 |
| Petrolatum | 4 |
| Silica | 1 |
| Palmöl | 4 |
| Polyethylen | 2 |
| Caprylic/Capric triglycerides | 10 |
| Isopropyl isostearate | 4 |
| Octyl dodecanol | 8 |
| Glyceryl monostearate | 2 |
| Rizinusöl | 12 |
| Glimmer | 4 |
| Reisstärke | 15 |
| Seidenpulver | 10 |
| Pigmente | 6 |

| **Phase B** | |
|---|---|
| Glycerin | 2 |

| Beispiel 8 | |
|---|---|
| **Innerer Kern** | |
| **Phase A** | |
| Carnaubawachs | 6 |
| Ozokerite | 19 |
| Oleyl alcohol | 49,7 |
| Propylene glycol | 2,3 |
| Rizinusöl | 5 |
| d-Iantrol | 5 |
| DC Red 21 | 0,4 |
| DC Red 7 lake | 0,6 |
| Black oxide | 1,1 |
| Ti-oxide | 2,5 |
| Red oxide | 2,3 |
| DC Red 6 | 0,1 |
| Propylene Glycol & Propyl Gallate & Citric Acid | 0,1 |
| EO PA 54675 | 0,9 |
| Oleyl alcohol | 5 |

### Arbeitsvorsvorschrift für alle Beispiele

Die Bestandteile der äußeren Umhüllung Phase A wurden in einen mit Dampf beheizten Mischbehälter nacheinander eingebracht und auf 80-85 °C erhitzt. Während der Erwärmung wurde gerührt. Nach dem Schmelzen der Phase A und einer gleichmäßigen Verteilung aller Bestandteile in dem Gemisch wurde die Phase B unter Rühren zugesetzt. Nachdem eine gleichmäßige Verteilung erreicht war, wurde das Gemisch in Formen für die äußere Umhüllung bei etwa 75 °C gegossen. Nach dem Abkühlen und nach Abziehen der Außenform wurde die Einsatzvorrichtung für den Innenkern herausgezogen und die vollständige Kristallisation der äußeren Umhüllung abgewartet. Anschließend wurde die Komposition des Innenkerns, die in ähnlicher Weise wie die der äußeren Umhüllung hergestellt worden war, in den Hohlraum der äußeren Umhüllung gegossen und der fertige Stift abgekühlt und anschließend komplettiert.

## Patentansprüche

1. Körperpflegemittel in Form eines frei stehenden Stiftes, **dadurch gekennzeichnet, daß** es aus einem Innenkern mit kosmetischen Bestandteilen und einer an dem Innenkern haftenden, festen äußeren Umhüllung besteht, wobei die äußere Umhüllung wachsfrei ist und wenigstens die folgenden Bestandteile enthält, bezogen auf die Zusammensetzung der äußeren Umhüllung,
(a) ein oder mehrere feste Proteine mit einem Anteil von 0,5 bis 50 Gew-%;
(b) ein oder mehrere flüssige Polyole oder eine Lösung von Polyolen mit einem Anteil von 0,2 bis 15 Gew-%;
(c) ein oder mehrere feste Polyole mit einem Anteil von 0,5 bis 80 Gew-%;
(d) ein oder mehrere Erweichungsmittel mit einem Anteil von 1 bis 90 Gew-%.

2. Körperpflegemittel nach Anspruch 1, **dadurch gekennzeichnet, daß** es weitere kosmetische Hilfsstoffe und aktive Ingredienzen mit einem Anteil von 0 bis 80 Gew-% enthält.

3. Körperpflegemittel nach Anspruch 1, **dadurch gekennzeichnet, daß** das Protein aus der Gruppe ausgewählt ist, bestehend aus Sojaprotein, Milchprotein, Weizenprotein, Seidenprotein, Erbsenprotein, Weizenprotein, Placentaprotein, Kollagen, Reisprotein, pflanzliches Protein, Weizengluten, Weizenkeimprotein, Molkenprotein, Kartoffelprotein, Süßmandelprotein, Reisschalenprotein, Hefeprotein, Keratin, Maisprotein, Markprotein, Conchiolin-Protein, Elastin, Serumprotein und Derivate davon, einschließlich hydrolysierte Derivate, Esterderivate, hydrierte Derivate sowie Gemische dieser Proteine.

4. Körperpflegemittel nach Anspruch 1, **dadurch gekennzeichnet, daß** der Anteil des festen Proteins im Bereich von 5 bis 50 Gew-% liegt.

5. Körperpflegemittel nach Anspruch 1, **dadurch gekennzeichnet, daß** der Anteil des festen Proteins im Bereich von 10 bis 50 Gew-% liegt, insbesondere im Bereich von 20 bis 40 Gew-% liegt.

6. Körperpflegemittel nach Anspruch 1, **dadurch gekennzeichnet, daß** der Anteil des festen Polyols im Bereich von 15 bis 70 Gew-% liegt.

7. Körperpflegemittel nach Anspruch 6, **dadurch gekennzeichnet, daß** der Anteil des festen Polyols im Bereich von 25 bis 70 Gew-% liegt, insbesondere im Bereich von 30 bis 70 Gew-%.

8. Körperpflegemittel nach Anspruch 6, **dadurch gekennzeichnet, daß** die flüssigen Polyole oder Lösungen von Polyolen für die äußere Umhüllung Substanzen sind, die geradkettige oder verzweigte Kohlenwasserstoffverbindungen mit wenigstens zwei Hydroxygruppen am Kohlenstoffgerüst einschließen.

9. Körperpflegemittel nach Anspruch 1, **dadurch gekennzeichnet, daß** die äußere Umhüllung oder der innere Kern weiterhin aktive Hautpflegesubstanzen, Antioxidationsmittel, UV-Filter, Feuchthaltemittel, Schutzmittel, Parfüm, Pigmente enthält.

10. Körperpflegemittel nach Anspruch 1, **dadurch gekennzeichnet, daß** die Bestandteile der äußeren Umhüllung als Gemisch einen Tropfpunkt von oberhalb 105 °C haben.

11. Körperpflegemittel nach Anspruch 1, **dadurch gekennzeichnet, daß** es ein Lippenstift ist.

## Claims

1. Personal care product in the form of a free-standing stick, comprising of an inner core with cosmetic ingredients and an solid outer shell adhering to the inner core, in which the outer shell is wax-free and contains at least the following ingredients, in relation to the composition of the outer shell,
(a) one or more solid proteins with a range of 0.5 to 50 weight %;
(b) one or more liquid polyols or solutions of polyols with a range of 0.2 to 15 weight %;
(c) one or more solid polyols with a range of 0.5 to 80 weight %;
(d) one or more emollients with a range from 1 to 90 weight %.

2. Personal care product according to claim 1, comprising additional cosmetic auxiliary materials and active ingredients with a range of between 0 and 80 weight %.

3. Product according to claim 1, wherein the protein is selected from the group consisting of soy protein, milk protein, oat protein, silk protein, pea protein, wheat protein, placental protein, collagen, rice protein, vegetable protein, wheat gluten, wheat germ protein, whey protein, potato protein, sweet almond protein, rice bran protein, yeast protein, keratin, corn protein, spinal protein, conchiorin protein, elastin, serum protein and their derivatives, which include hydrolyzed derivatives, ester derivatives, hydrogenated derivatives; and mixtures thereof.

4. Product according to claim 1, wherein the amount of the solid protein is in the range of 5 to 50 % by weight.

5. Product according to claim 1, wherein the amount of the solid protein is in the range of 10 to 50 % by weight, especially in the range of 20 to 40 % by weight.

6. Product according to claim 1, wherein the amount of the solid polyol is in the range of 15 to 70 % by weight.

7. Product according to claim 6, wherein the amount of the solid polyol is in the range of 25 to 70 % by weight, especially in the range of 30 to 70 % by weight.

8. Product according to claim 6, wherein the liquid polyols or solutions of polyols for the outer shell are materials which include straight or branched chained hydrocarbon compounds containing at least two hydroxyl groups on the carbon skeleton.

9. Product according to claim 1, wherein the outer shell, the inner core or both contains active skin-care ingredients, anti-oxidation agents, UV filters, moisturising agents, protective agents, perfume, pigments.

10. Product according to claim 1, wherein the ingredients of the outer shell as mixture have a drop melting point of above 105 °C.

11. Product according to claim 1 wherein it is a lip stick.

## Revendications

1. Produit d'hygiène corporelle en forme de bâton autoportant, **caractérisé en ce qu'**il se compose d'un noyau interne avec des constituants cosmétiques et d'une enveloppe externe solide, adhérant au noyau interne, l'enveloppe externe étant exempte de cire et contenant au moins les constituants suivants, par rapport à la composition de l'enveloppe externe,
(a) une ou plusieurs protéines solides dans une proportion de 0,5 à 50 % en poids ;
(b) un ou plusieurs polyols liquides ou une solution de polyols dans une proportion de 0,2 à 15 % en poids ;
(c) un ou plusieurs polyols solides dans une proportion de 0,5 à 80 % en poids ;
(d) un ou plusieurs agents de ramollissement dans une proportion de 1 à 90 % en poids.

2. Produit d'hygiène corporelle selon la revendication 1, **caractérisé en ce qu'**il contient des adjuvants cosmétiques supplémentaires et des ingrédients actifs dans une proportion de 0 à 80 % en poids.

3. Produit d'hygiène corporelle selon la revendication 1, **caractérisé en ce que** la protéine est choisie dans le groupe constitué par la protéine de soja, la protéine de lait, la protéine de blé, la protéine de soie, la protéine de pois vert, la protéine de blé, la protéine de placenta, le collagène, la protéine de riz, la protéine végétale, le gluten de blé, la protéine de germe de blé, la protéine de petit-lait, la protéine de pomme de terre, la protéine d'amande douce, la protéine d'enveloppe de riz, la protéine de levure, la kératine, la protéine de maïs, la protéine de moelle, la protéine de conchioline, l'élastine, la protéine de sérum et des dérivés de ceux-ci, y compris des dérivés hydrolysés, des dérivés d'ester, des dérivés hydrogénés ainsi que des mélanges de ces protéines.

4. Produit d'hygiène corporelle selon la revendication 1, **caractérisé en ce que** la proportion de la protéine solide se trouve dans la plage de 5 à 50 % en poids.

5. Produit d'hygiène corporelle selon la revendication 1, **caractérisé en ce que** la proportion de la protéine solide se trouve dans la plage de 10 à 50 % en poids, en particulier dans la plage de 20 à 40 % en poids.

6. Produit d'hygiène corporelle selon la revendication 1, **caractérisé en ce que** la proportion du polyol solide se trouve dans la plage de 15 à 70 % en poids.

7. Produit d'hygiène corporelle selon la revendication 6, **caractérisé en ce que** la proportion du polyol solide se trouve dans la plage de 25 à 70 % en poids, en particulier dans la plage de 30 à 70 % en poids.

8. Produit d'hygiène corporelle selon la revendication 6, **caractérisé en ce que** les polyols liquides ou les solutions de polyols pour l'enveloppe externe sont des substances, qui contiennent des composés hydrocarbonés linéaires ou ramifiés ayant au moins deux groupes hydroxyles sur le squelette carboné.

9. Produit d'hygiène corporelle selon la revendication 1,
**caractérisé en ce que** l'enveloppe externe ou le noyau interne contient en outre des substances d'hygiène cutanée actives, des agents anti-oxydation, des filtres UV, des agents humectants, des agents protecteurs, du parfum, des pigments.

10. Produit d'hygiène corporelle selon la revendication 1, **caractérisé en ce que** les composants de l'enveloppe externe ont en tant que mélange un point de goutte supérieur à 105°C.

11. Produit d'hygiène corporelle selon la revendication 6, **caractérisé en ce que** c'est un bâton de rouge à lèvres.
